(19) Europäisches Patentamt
European Patent Office
Office européen des brevets

(11) **EP 4 407 633 A1**

(12) **EUROPEAN PATENT APPLICATION**
published in accordance with Art. 153(4) EPC

(43) Date of publication:
**31.07.2024 Bulletin 2024/31**

(21) Application number: **22873142.8**

(22) Date of filing: **15.09.2022**

(51) International Patent Classification (IPC):
*G16H 50/70* (2018.01)      *G16H 50/50* (2018.01)
*G16H 50/20* (2018.01)      *G16H 10/60* (2018.01)
*A61B 5/28* (2021.01)      *G06N 3/08* (2023.01)

(52) Cooperative Patent Classification (CPC):
**A61B 5/28; G06N 3/08; G16H 10/60; G16H 50/20; G16H 50/50; G16H 50/70**

(86) International application number:
**PCT/KR2022/013833**

(87) International publication number:
**WO 2023/048437 (30.03.2023 Gazette 2023/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.09.2021 KR 20210126784**
**08.09.2022 KR 20220113975**

(71) Applicant: **Medical AI Co., Ltd.**
**Seoul 06302 (KR)**

(72) Inventors:
• **KWON, Joonmyoung**
**Seoul 06302 (KR)**
• **LEE, Byeongtak**
**Seoul 06302 (KR)**

(74) Representative: **Patentanwaltskanzlei**
**Matschnig & Forsthuber OG**
**Biberstraße 22**
**Postfach 36**
**1010 Wien (AT)**

(54) **METHOD, PROGRAM, AND APPARATUS FOR TRAINING AND INFERRING DEEP LEARNING MODEL ON BASIS OF MEDICAL DATA**

(57)    According to an embodiment of the present disclosure, there are disclosed a method, program, and device for the training and inference of a deep learning model based on medical data, which are performed by a computing device. The training method includes: training a first neural network model based on medical data; and training a second neural network model based on the trained first neural network model by matching a first operation function representative of a neural network block included in the trained first neural network model and a second operation function representative of a neural network block included in the second neural network model.

FIG. 2

**Description**

**Technical Field**

The present disclosure relates to deep learning technology in the medical field, and more particularly to a method of training a deep learning model by using inductive bias transfer and utilizing the trained deep learning model in the medical field.

**Background Art**

In order to generalize a deep learning model, there is required the use of a large amount of data. For example, one of the conventional transformer models achieves higher performance than other convolution-based approaches on the ImageNet benchmark using 300 million additional data points. However, it is not easy to secure big data in all domains. Accordingly, the use of a deep learning model is still limited in domains where acquiring a large amount of data is considerably costly, such as the medical field.

Many researchers are proposing solutions that alleviate the problem of generalizing a deep learning model without relying on a massive amount of data. One of the solutions is Data efficient image Transformer (DeiT) based on knowledge distillation. DeiT adopts a method of distilling the knowledge of a convolutional neural network, which plays an important role in generalization performance, into a transformer through additional tokens by using the convolutional neural network as a teacher for the transformer. DeiT assumes that performance can be improved by encoding the inductive bias of the convolutional neural network into the transformer through the distillation method.

Nevertheless, in order to perform knowledge distillation, DeiT also requires a predetermined amount of data to be secured. Accordingly, DeiT has the problem of being difficult to utilize in the medical field in which a sufficient number of data classes for knowledge distillation have not been accumulated. For example, ImageNet, which has been previously used to evaluate the performance of DieT, consists of thousands of classes, whereas medical data such as electrocardiogram data, electronic health records (EHR) data, and/or the like contain only tens to tens of classes. When the fact that successful knowledge transfer depends on the signal quality of a teacher to which various signals are important is taken into consideration, it is obvious that when DieT is used, the inductive bias of a teacher model is successfully transferred in the case of ImageNet, but is not transferred in the case of medical data.

**Disclosure**

**Technical Problem**

The present disclosure has been conceived in response to the above-described background technology, and an object of the present disclosure is to provide a method of training a deep learning model by efficiently transferring inductive bias even using a small data set in the medical field and also provide a method of estimating various health conditions by using a deep learning model trained based on the above training method.

However, the objects to be accomplished by the present disclosure are not limited to the object mentioned above, and other objects not mentioned may be clearly understood based on the following description.

**Technical Solution**

According to an embodiment of the present disclosure for accomplishing the above-described object, there is disclosed a method of training a deep learning model based on medical data, which is performed by a computing device. The training method may include: training a first neural network model based on medical data; and training a second neural network model based on the trained first neural network model by matching a first operation function representative of a neural network block included in the trained first neural network model and a second operation function representative of a neural network block included in the second neural network model.

Alternatively, training the second neural network model based on the trained first neural network model by matching the first operation function representative of the neural network block included in the trained first neural network model and the second operation function representative of the neural network block included in the second neural network model may include training the second neural network model based on the trained first neural network model by using a loss function for matching at least one of inputs or dimensions between the first and second operation functions.

Alternatively, the loss function may include: a first sub-loss function adapted to use th output of a first operation function corresponding to an (n-1)-th (n is a natural number) neural network block included in the first neural network model as an input variable; and a second sub-loss function adapted to use the output of a second operation function corresponding to an (n-1)-th neural network block included in the second neural network model as an input variable. In this case, each of the first and second sub-loss functions may be a function for calculating the difference between the output of a first operation function corresponding to an n-th neural network block included in the first neural network model and the output of a second operation function corresponding to an n-th neural network block included in the second neural network model.

Alternatively, each of the first and second sub-loss functions may include a transformation function for matching the dimensions of the first operation function corresponding to the n-th neural network block and the second operation function corresponding to the n-th neural network block.

Alternatively, the transformation function may include: a first sub-transformation function for linearly transforming the input variables of the transformation function in a temporal direction; and a second sub-transformation function for linearly transforming the input variables of the transformation function in a feature dimension.

Alternatively, the transformation function included in the first sub-loss function may be a function for: matching the dimension of the output of the first operation function corresponding to the (n-1)-th neural network block to the dimension of the input of the second operation function corresponding to the n-th neural network block; and matching the dimension of the output of the second calculation function corresponding to the n-th neural network block to the dimension of the output of the first calculation function corresponding to the n-th neural network block.

Alternatively, the transformation function included in the second sub-loss function may be a function for: matching the dimension of the output of the second operation function corresponding to the (n-1)-th neural network block to the dimension of the input of the first operation function corresponding to the n-th neural network block; and matching the dimension of the output of the first operation function corresponding to the n-th neural network block to the dimension of the output of the second operation function corresponding to the n-th neural network block.

Alternatively, the loss function may further include a third sub-loss function for calculating the difference between the output of the first neural network model having received the medical data and the output of the second neural network model having received the medical data.

Alternatively, the third sub-loss function may include a transformation function for matching the dimension of the output of the first neural network model having received the medical data to the dimension of the output of the second neural network model having received the medical data.

Alternatively, the method may further include fine-tuning the second neural network model based on the medical data. In this case, the fine-tuning may be training the second neural network model while maintaining the weight of the second neural network model close to a weight in the state in which training based on the trained first neural network model has been completed.

Alternatively, the first neural network model may include at least one of a convolutional neural network or a recurrent neural network, and the second neural network model may include a self-attention-based neural network.

Alternatively, the medical data may include at least one of electrocardiogram data or electronic health records (EHR) data.

Meanwhile, according to an embodiment of the present disclosure for accomplishing the above-described object, there is disclosed an inference method of a deep learning model based on medical data, which is performed by a computing device. The inference method may include: acquiring medical data including at least one of electrocardiogram data or electronic health records (EHR) data; and estimating a person's health condition based on the medical data by using a second neural network model. In this case, the second neural network model has been trained through operations for matching a first operation function corresponding to a neural network block included in a pre-trained first neural network model and a second operation function corresponding to a neural network block included in the second neural network model based on the pre-trained first neural network model.

According to an embodiment of the present disclosure for accomplishing the above-described object, there is disclosed a computer program stored in a computer-readable storage medium. The computer program performs operations for training a deep learning model based on medical date when executed on one or more processors. In this case, the operations may include operations of: training a first neural network model based on medical data; and training a second neural network model based on the trained first neural network model by matching a first operation function representative of a neural network block included in the trained first neural network model and a second operation function representative of a neural network block included in the second neural network model.

According to an embodiment of the present disclosure for accomplishing the above-described object, there is disclosed a computing device for training a deep learning model based on medical data. The computing device may include: a processor including at least one core; memory including program codes that are executable on the processor; and a network unit configured to acquire medical data. The processor may train a first neural network model based on medical data, and may train a second neural network model based on the trained first neural network model by matching a first operation function representative of a neural network block included in the trained first neural network model and a second operation function representative of a neural network block included in the second neural network model.

**Advantageous Effects**

The present disclosure provides the method of training a deep learning model, which can generalize a student model even based on a small amount of data by appropriately utilizing the strong inductive bias of a teacher model, and also provides the method of effectively utilizing a deep learning model, trained through the above training method, in the medical field.

**Description of Drawings**

FIG. 1 is a block diagram of a computing device according to an embodiment of the present disclosure;
FIG. 2 is a block diagram showing a process of training a deep learning model according to an embodiment of the present disclosure;
FIG. 3 is a conceptual diagram showing the operation process of a loss function according to an embodiment of the present disclosure;
FIG. 4 is a graph showing the results of the comparison and evaluation of the performance of a deep learning model trained according to an embodiment of the present disclosure and the performance of a conventional solution;
FIG. 5 is a flowchart showing a method of training a deep learning model according to an embodiment of the present disclosure; and
FIG. 6 is a flowchart showing the inference method of a deep learning model according to an embodiment of the present disclosure.

**Mode for Invention**

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "acquisition" used herein can be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

"Data" used herein may include "images," signals, etc. The term "image" used herein may refer to multidimensional data composed of discrete image elements. In other words, "image" may be understood as a term referring to a digital representation of an object that can be seen by the human eye. For example, "image" may refer to multidimensional data composed of elements corresponding to pixels in a two-dimensional image. "Image" may refer to multidimensional data composed of elements corresponding to voxels in a three-dimensional image.

The term "inductive bias" used herein can be understood as a set of assumptions that enable the inductive inference of a deep learning model. In order to resolve the generalization error in which a deep learning model exhibits appropriate performance only for given training data, it is necessary for the deep learning model to infer an output close to an accurate output for data other than the given training data. Accordingly, "inductive bias" can be understood as a set of preconditions that a deep learning model has in the process of being trained to predict the output of an ungiven input.

The term "block" used herein may be understood as a set of components classified based on various criteria such as type, function, etc. Accordingly, the components classified as each "block" may be changed in various manners depending on the criteria. For example, a neural network "block" may be understood as a set of neural networks including one or more neural networks. In this case, it can be assumed that the neural networks included in the neural network "block" perform predetermined operations to achieve a common purpose that serves as a classification criterion.

The term "operation function" used herein can be understood as a mathematical presentation for a configurational unit that performs a specific function or processes an operation. For example, the "operation function" of a neural network block can be understood as a mathematical representation representative of a neural network block that processes a specific operation. Accordingly, the relationship between the input and output of the neural network block can be represented by a formula through the "operation function" of the neural network block.

The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

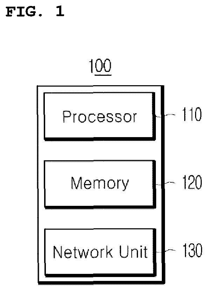

FIG. 1 is a block diagram of a computing device according to an embodiment of the present disclosure.

A computing device 100 according to an embodiment of the present disclosure may be a hardware device or part of a hardware device that performs the comprehensive processing and calculation of data, or may be a software-based computing environment that is connected to a communication network. For example, the computing device 100 may be a server that performs an intensive data processing function and shares resources, or may be a client that shares resources through interaction with a server. Furthermore, the computing device 100 may be a cloud system in which a plurality of servers and clients interact with each other and comprehensively process data. Since the above descriptions are only examples related to the type of computing device 100, the type of computing device 100 may be configured in various manners within a range understandable to those skilled in the art based on the content of the present disclosure.

Referring to FIG. 1, the computing device 100 according to an embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to an embodiment of the present disclosure may be understood as a configuration unit including hardware and/or software for performing computing operation. For example, the processor 110 may read a computer program and perform data processing for machine learning. The processor 110 may process computational processes such as the processing of input data for machine learning, the extraction of features for machine learning, and the calculation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may train a neural network model for estimating a person's health condition based on knowledge distillation, which transfers the knowledge of a teacher model to a student model. For example, the processor 110 may train the teacher model to estimate a person's health condition based on medical data. The processor 110 may train the student model based on the inductive bias generated during the process of training the teacher model. In order to transfer the inductive bias of the teacher model to the student model during the process of training the student model, the processor 110 may match individual operation functions representative of a plurality of neural network blocks included in the teacher model and individual operation functions representative of a plurality of neural network blocks included in the student model. In this case, the matching of the operation functions may be understood as an operation that allows the student model to be trained on the inductive bias of the teacher model by minimizing the difference between the features interpreted by the individual neural network blocks of the teacher model and the features interpreted by the individual neural network blocks of the student model. Through the matching of operation functions, the processor 110 may effectively transfer the inductive bias of the teacher model to the student model even by using a small amount of medical data. In other words, through the training for the performance of the matching of operation functions, the processor 110 may efficiently train the student model based on the knowledge of the teacher model even when a sufficient amount of data is not secured. Additionally, through the training for the performance of the matching of operation functions, the processor 110 may guarantee the performance of generalization of the student model.

The processor 110 may estimate a person's health condition based on medical data by using the neural network model generated through the above-described training process. The processor 110 may generate inference data representative of the results of estimation of a person's health condition by inputting medical data to the neural network model trained through the above-described process. For example, the processor 110 may predict whether a chronic disease such as arrhythmia has occurred and the progress of the chronic disease by inputting electrocardiogram data to the trained student model. The processor 110 may generate data related to information necessary for the management of a patient, such as changes in the patient's heart rate by inputting electronic health records (EHR) data to the trained student model. In addition to the examples described above, the type of medical data and the output of the neural network model may be configured in various manners within a range understandable to those skilled in the art based on the content of the present disclosure.

The memory 120 according to an embodiment of the present disclosure may be understood as a configuration unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data necessary for the processor 110 to perform computation, the combination of data, and program codes executable on the processor 110. For example, the memory 120 may store medical data received through the network unit 130, which will be described later. The memory 120 may store program codes configured to operate the neural network model to receive medical data and perform learning, program codes configured to operate the neural network model to receive medical data and perform inference in accordance with the purpose of use of the computing device 100, processed data generated as program codes are executed, etc.

The network unit 130 according to an embodiment of the present disclosure may be understood as a configuration unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wideband wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data necessary for the processor 110 to perform computation through wired/wireless communication with any system or client or the like. Furthermore, the network unit 130 may transmit data generated through the computation of the processor 110 through wired/wireless communication with any system or client or the like. For example, the network unit 130 may receive medical data through communication with a database within a hospital environment, a cloud server configured to perform tasks such as the standardization of medical data, a computing device, or the like. The network unit 130 may transmit the output data of the neural network model, intermediate data and processed data acquired from the computation process of the processor 110, etc. through communication with the above-described database, server, or computing device.

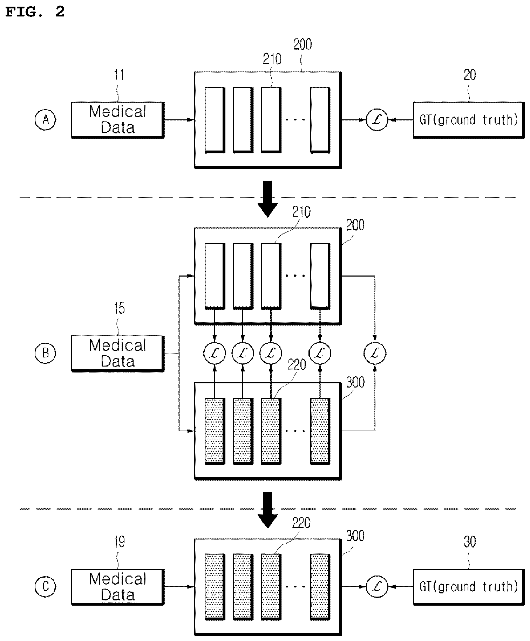

FIG. 2 is a block diagram showing a process of training a deep learning model according to an embodiment of the present disclosure.

Referring to FIG. 2, the processor 110 according to an embodiment of the present disclosure may perform a three-step training process to train a neural network model for estimating a person's health condition based on medical data. The processor 110 may perform step A of training a first neural network model 200 to have an inductive bias for estimating a person's health condition based on medical data. The processor 110 may perform step B of transferring the inductive bias of the first neural network model 200, enhanced through step A, to the second neural network model 300. Furthermore, the processor 110 may perform step C of fine-tuning the second neural network model 300, trained through step B, to suit the purpose of use. However, step C does not necessarily need to be performed, and may or may not be performed depending on the purpose of using the second neural network model 300.

More specifically, the processor 110 may train the first neural network model 200 to generate an inductive bias to be transferred to the second neural network model 300. The processor 110 may generate an output for estimating a person's health condition by inputting medical data 11 to the first neural network model 200. The processor 110 may reconstruct the parameters, such as the weight, of the neural network by comparing the output of the first neural network model 200 with the ground truth (GT) through a loss function. The processor 110 may train the first neural network model 200 based on the medical data 11 by repeatedly performing the above operation until the loss function converges. When the first neural network model 200 includes a convolutional neural network, the first neural network model 200 may enhance the inductive bias of locality and translation invariance for the medical data 11 through step A. When the first neural network model 200 includes a recurrent neural network, the first neural network model 200 may enhance the inductive bias of temporal invariance for the medical data 11 through step A.

The processor 110 may match a plurality of neural network blocks 210 included in the first neural network model 200 with a plurality of neural network blocks 220 included in the second neural network model 300 so that the second neural network model 300 can be trained on the inductive bias of the first neural network model 200 enhanced through step A. The processor 110 may input medical data 15 to each of the first neural network model 200 and the second neural network model 300. In this case, the medical data 15 of step B may be the same as or different from the medical data 11 of step A. In the process in which the individual models 200 and 300 process the medical data 15, the processor 100 may calculate the difference between the output of each of the plurality of neural network blocks 210 included in the first neural network model 200 and the output of each of the plurality of neural network blocks 220 included in the second neural network model 300 by using the loss function. That is, the processor 110 may pair the plurality of neural network blocks 210 included in the first neural network model 200 and the plurality of neural network blocks 220 included in the second neural network model 300 and then calculate loss.

However, due to the types of neural networks included in the first and second neural network models 200 and 300 and the dependency between the neural networks, the differences in the input and/or dimension may occur between the neural network blocks 210 and 220 to be matched. When the first neural network model 200 includes at least one of a convolutional neural network or a recurrent neural network and the second neural network model 300 includes a self-attention-based neural network, it is inevitably difficult to calculate the loss due to the differences in the input and dimension between the neural network blocks 210 of the first neural network model 200 and the neural network blocks 220 of the second neural network model 300. Accordingly, the processor 110 may perform an operation of matching the inputs and/or dimensions between the neural network blocks 210 and 220 in the process of calculating the differences using the loss function. For example, the processor 110 may alternately input the intermediate output of the first neural network model 200 and the intermediate output of the second neural network model 300 to the blocks 210 and 220, and may perform an operation of matching the input and output dimensions of the blocks 210 and 220. Through such input and dimension matching, the processor 110 may effectively match the neural network blocks 210 and 220 even when the types of neural networks included in the first neural network model 200 and the second neural network model 300 are different.

In addition, the processor 110 may calculate the difference between the final output of the first neural network model 200 and the final output of the second neural network model 300 by using a loss function. The processor 110 may transfer the inductive bias of the first neural network model 200 to the second neural network model 300 by repeatedly performing the above-described matching between the neural network blocks and the above-described matching between the final outputs until the loss function converges. In other words, the processor 110 may train the second neural network model 300 based on the first neural network model 200, trained through step A, through the neural network block matching in step B.

Once the above-described step B has been completed, the processor 110 may fine-tune the second neural network model 300 that has been trained on the inductive bias of the first neural network model 200. In this case, the fine tuning may be understood as a training process of optimizing the second neural network model 300 for a specific task in accordance with the purpose of use of the second neural network model 300. The processor 110 may generate an output for estimating the person's health condition by inputting the medical data 19 to the second neural network model 300 trained through step B. In this case, the medical data 19 may be electrocardiogram data or electronic health records data depending on the purpose of using the second neural network model 300. Additionally, the second neural network model 300 may receive electrocardiogram data to estimate chronic disease such as arrhythmia, and may receive electronic health records data to estimate changes in the condition of a critically ill patient. However, since the input and output of the second neural network model 300 are only examples, the present disclosure is not limited to the above-described examples. The processor 110 may reconstruct the parameters, such as the weight, of the neural network by comparing the output of the second neural network model 300 with the ground truth GT through the loss function. The processor 110 may re-train the second neural network model 300 based on the medical data 19 by repeatedly performing the above operation until the loss function converges.

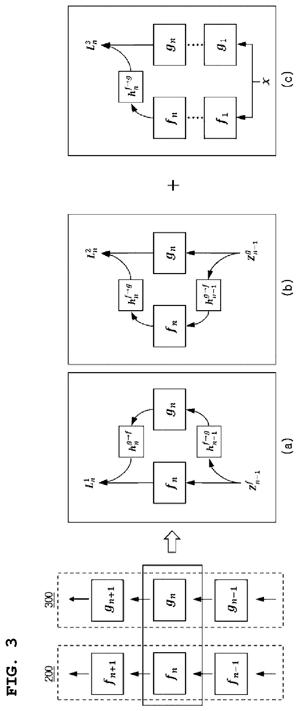

FIG. 3 is a conceptual diagram showing the operation process of a loss function according to an embodiment of the present disclosure. In the following description for FIG. 3, the first neural network model 200 is assumed to be a convolutional neural network model or a recurrent neural network model, and the second neural network model 300 is assumed to be a self-attention-based transformer model.

Referring to FIG. 3, the first neural network model 200 and the second neural network model 300 may be interpreted as a combination of operation functions representative of neural network blocks as shown in Equation 1 below.

$$f = l_f \circ f_n \circ \cdots \circ f_1$$
$$g = l_g \circ g_n \circ \cdots \circ g_1 \tag{1}$$

where $f$ denotes the first neural network model 200, $f_n$ denotes the n-th (n is a natural number) neural network block of the first neural network model 200, $g$ denotes the second neural network model 300, and $g_n$ denotes the n-th neural network block of the second neural network model 300. Furthermore, $l_f$ denotes the classifier of the first neural network model 200, and $l_g$ denotes the classifier of the second neural network model 300.

In order to transfer the inductive bias of the first neural network model 200, which is a convolutional neural network model or a recurrent neural network model, to the second neural network model 300, which is a transformer model, the processor 110 according to an embodiment of the present disclosure may perform an operation of matching $f_n$ and $g_n$. Matching operations may be basically divided into input matching and dimension matching.

Due to the difference in the types of neural networks between the first and second neural network models 200 and 300, problems with an input difference may naturally occur in function matching between neural network blocks. For example, the input of the second function $g_2$ of the second neural network model 300 is $g_1(x)$, and the input of the second function $f_2$ of the first neural network model 200 is $f_1(x)$. In this case, when input matching is not performed, matching between the two functions is performed as an operation of minimizing $\|g_2(g_1(x)) - f_2(f_1(x))\|$. However, due to the dependency of previous neural network blocks, this matching operation may not guarantee that $g_2$ is close to $f_2$. Accordingly, the processor 110 alleviates the above-described problem by transferring signals to both operation flows of the first neural network model 200 and the second neural network model 300. That is, in the present disclosure, matching between the two functions is performed as an operation of minimizing $\|g_2(f_1(x)) - f_2(f_1(x))\| - \|g_2(g_1(x)) - f_2(g_1(x))\|$.

Furthermore, in function matching between neural network blocks, a problem with a dimensional difference may occur due to the functions through which input passes. In the case of $f_n(\cdot)$ representative of the n-th neural network block of the first neural network model 200, a pooling operation and a convolution-with-stride operation change a dimension according to a temporal direction, and a convolution operation change also expands the dimension of features. To handle this problem, the processor 110 may use a transformation function $h(\cdot)$ that transforms individual dimensions into the same one.

$$h(z) = I(z)W \tag{2}$$

In Equation 2, $I(\cdot)$ denotes the linear transformation of the temporal direction, and $W$ denotes the linear transformation of the feature dimension. In other words, the transformation function $h(\cdot)$ may be represented by the product of a function for the linear transformation of the temporal direction of an input variable and a function for the linear transformation of the feature dimension of the input variable.

To sum up the input matching and dimension matching described above, they may be represented by two loss functions as shown in Equation 3 below:

$$L_n^1 = \sum_{t,d}\big(f_n(z_{n-1}^f) - (h_n^{g\to f} \circ g_n \circ h_{n-1}^{f\to g})(z_{n-1}^f)\big)^2$$

$$L_n^2 = \sum_{t,d}\big(g_n(z_{n-1}^g) - (h_n^{f\to g} \circ f_n \circ h_{n-1}^{g\to f})(z_{n-1}^g)\big)^2 \qquad (3)$$

where $L_n^1$ may correspond to (a) of FIG. 3, and $L_n^2$ may correspond to (b) of FIG. 3.

Furthermore, referring to (c) of FIG. 3, in order to allow not only the operation function $g_n$ corresponding to the neural network block of the second neural network model 300 but also the overall g of the second neural network model 300 to receive the inductive bias of the first neural network model 200 and operate similarly, the operation of the loss function $L_n^3$ shown in Equation 4 below may be additionally performed.

$$L_n^3 = \sum_{t,d}((f_1 \circ \cdots \circ f_n)(x) - (g_1 \circ \cdots \circ g_n)(x))^2 \qquad (4)$$

The total loss function L for training the second neural network model 300, which is a transformer model, on the inductive bias of the first neural network model 200, which is a convolutional neural network model or a recurrent neural network model, may be represented by the following Equation 5 obtained by integrating the above-described Equations 3 and 4.

$$L = \sum_n(L_n^1 + L_n^2 + L_n^3)$$

$$\varnothing_{f\to g}^* = argmin_{\varnothing_{f\to g}} \sum_{x\in D} L\left(g(x;\varnothing_{f\to g}), f(x;\varnothing_f^*)\right) \qquad (5)$$

Meanwhile, once the training of the second neural network model 300 based on the above-described loss function has been completed according to an embodiment of the present disclosure, the weight Ø in the state in which training has been completed may be used as the initial weight of training using a randomly initialized classifier. The processor 110 may fine-tune the trained second neural network model 300 by minimizing loss through the same task as the first neural network model 200 having an inductive bias.

While the second neural network model 300 is being fine-tuned, the processor 110 may normalize the second neural network model 300 so that the second neural network model 300 can be prevented from forgetting the inductive bias, transferred from the first neural network model 200, by keeping the weight $\varnothing_g$ close to the initial weights $\varnothing_{f\to g}$.

This normalization may be represented by Equation 6 below:

$$L_{L2-reg} = \left\|\varnothing_{f\to g}^* - \varnothing_g\right\|_2^2$$

$$\varnothing_g^* = argmin_{\varnothing_g} \sum_{(x,y)\in D} L\left(g(x;\varnothing_g, y)\right) + L_{L2-reg} \qquad (6)$$

Through the three-step training of FIG. 2 and the operation of the loss function of FIG. 3 described above, training is performed by efficiently transferring the inductive bias of the first neural network model 200 to the second neural network model 300, and the second neural network model 300 may be effectively optimized to suit the purpose of use in the medical field. Furthermore, in the medical field in which sufficient data is not accumulated, the second neural network model 300 may be trained to ensure generalization performance even by using a small amount of data, so that it can be easily used for various tasks in the medical field.

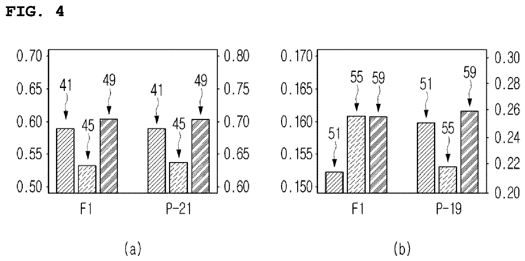

FIG. 4 is a graph showing the results of the comparison and evaluation of the performance of a deep learning model trained according to an embodiment of the present disclosure and the performance of a conventional solution.

FIG. 4(a) shows the results of the comparison among the performance of the convolutional neural network 41, the performance of a DieT 45, and the performance of the neural network 49 of the present disclosure, trained through the reception of the inductive bias from the convolutional neural network 41, based on electrocardiogram data. Furthermore, F1 represents an f-1 score, and P-21 represents a physionet 21 score. In the case of the electrocardiogram data, it can be seen that the performance of the neural network 49 of the present disclosure is superior to that of the convolutional neural network 41 that has transferred the inductive bias. Furthermore, it can be seen that the performance of the neural network 49 of the present disclosure is significantly superior to that of the DieT 45, which is based on knowledge distillation. Particularly in the electrocardiogram case, it can be seen that the performance of the DieT 45 is inferior to that of the convolutional neural network 41.

FIG. 4(b) shows the results of the comparison among the performance of the convolutional neural network 51, the performance of a DieT 55, and the performance of the neural network 59 of the present disclosure, trained through the reception of the inductive bias from the convolutional neural network 51, based on electronic medical record data. Furthermore, P-19 represents a physionet 19 score. In the case of the electronic medical record data, it can be seen that the performance of the neural network 59 of the present disclosure is superior to that of the convolutional neural network 51 that has transferred the inductive bias. Furthermore, it can be seen that the performance of the neural network 49 of the present disclosure is significantly superior to that of the DieT 45, which is based on knowledge distillation, based on P-15.

To sum up the above-described results, it can be seen that the neural network trained through the training method of the present disclosure is more suitable for the medical field in which sufficient data for training is not accumulated than the DieT. Furthermore, it can be seen that the neural network trained through the training method of the present disclosure successfully encodes the inductive bias of a preceding model and exhibits better performance than the preceding model.

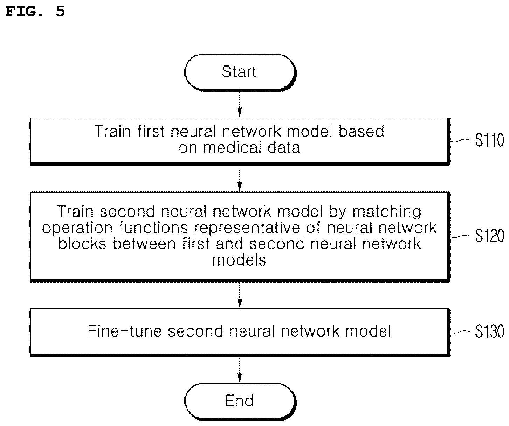

FIG. 5 is a flowchart showing a method of training a deep learning model according to an embodiment of the present disclosure.

Referring to FIG. 5, the computing device 100 according to an embodiment of the present disclosure may train a first neural network model based on medical data in step S110. The computing device 100 may train the first neural network model so that the first neural network model can estimate the health condition of a person based on the medical data. For example, when the computing device 100 is a server or client of a cloud system, the computing device 100 may receive at least one of electrocardiogram data or electronic health records data as training data through communication with a database in a hospital environment. When the computing device 100 is a database within a hospital environment, the computing device 100 may generate at least one of electrocardiogram data or electronic health records data as training data through communication with an electrocardiogram measurement device within a hospital environment. The computing device 100 may train the first neural network model to estimate the occurrence, change trend, degree of risk, and/or the like of a human disease based on at least one of the electrocardiogram data or electronic health records data obtained by the first neural network model.

The computing device 100 may train the second neural network model by matching operation functions representative of neural network blocks between the first and second neural network models in step S120. The computing device 100 may match a first operation function representative of a neural network block included in the first neural network model trained through step S110 and a second operation function representative of a neural network block included in the second neural network model. More specifically, the computing device 100 may train the second neural network model based on the first neural network model trained through step S110 by using a loss function for matching at least one of the inputs or dimensions between the first operation function and the second operation function.

According to an embodiment of the present disclosure, the loss function for matching the first and second operation functions in step S120 may include a first sub-loss function whose input variable is the output of the first operation function corresponding to an (n-1)-th neural network block included in the first neural network model, and a second sub-loss function whose input variable is the output of the second operation function corresponding to an (n-1)-th neural network block included in the second neural network model. Each of the first and second sub-loss functions may be a function for calculating the difference between the output of the first operation function corresponding to the n-th neural network block included in the first neural network model and the output of the first operation function corresponding to the n-th neural network block included in the second neural network model. That is, the first sub-loss function may be understood as a function for calculating the difference between the outputs generated by inputting the output of the previous first operation function to the current first and second operation functions. Additionally, the second sub-loss function may be understood as a function for calculating the difference between the outputs generated by inputting the output of the previous second operation function to the current first and second operation functions.

When the first and second neural network models include the same convolutional neural network blocks, a data dimension problem does not occur during a process in which each operation function performs processing. However, when the first neural network model includes convolutional neural network blocks and the second neural network model includes a different type of neural network blocks as in the case where it includes self-attention-based neural network blocks, a data dimension problem may occur during a process in which each operation function performs processing. Accordingly, each of the first and second sub-loss functions may include a transformation function for matching the dimension of the first operation function corresponding to the n-th neural network block and the dimension of the second operation function corresponding to the n-th neural network block. In this case, the transformation function may include a first sub-transformation function for linearly transforming the input variable of the transformation function in the temporal direction and a second sub-transformation function for linearly transforming the input variable of the transformation function in the feature dimension. It may be understood that the transformation function corresponds to $h(\cdot)$ in Equation 2, the first sub-transformation function corresponds to $l(\cdot)$ in Equation 2 and the second sub-transformation function corresponds to $W$ in Equation 2.

The transformation function included in the first sub-loss function may be a function for matching the dimension of the output of the first operation function corresponding to the (n-1)-th neural network block to the dimension of the input of the second operation function corresponding to the n-th neural network block and also matching the dimension of the output of the second calculation function corresponding to the n-th neural network block to the dimension of the output of the first operation function corresponding to the n-th neural network block. For example, referring to (a) of FIG. 3, the transformation function included in the first sub-loss function may be used for an operation for transforming the dimension of the output of the first operation function corresponding to the (n-1)-th neural network block in accordance with the dimension of the input of the second operation function corresponding to the n-th neural network block, as in

$$h_{n-1}^{f \to g}$$

. Furthermore, the transformation function included in the first sub-loss function may be used for an operation of transforming the dimension of the output of the second operation function corresponding to the n-th neural network block in accordance with the dimension of the output of the first operation function corresponding to the n-th neural network block, as in $$h_{n}^{g \to f}$$ .

The transformation function included in the second sub-loss function may be a function for matching the dimension of the output of the second operation function corresponding to the (n-1)-th neural network block to the dimension of the input of the first operation function corresponding to the n-th neural network block and also matching the dimension of the output of the first operation function corresponding to the n-th neural network block to the dimension of the output of the second operation function corresponding to the n-th neural network block. For example, referring to (b) of FIG. 3, the transformation function included in the second sub-loss function may be used for an operation of transforming the dimension of the output of the second operation function corresponding to the (n-1)-th neural network block in accordance with the dimension of the input of the first operation function corresponding to the n-th neural network block, as in $$h_{n-1}^{g \to f}$$ . Furthermore, the transformation function included in the second sub-loss function may be used for an operation of transforming the dimension of the output of the first operation function corresponding to the n-th neural network block in accordance with the dimension of the output of the second operation function corresponding to the n-th neural network block, as in $$h_{n}^{f \to g}$$ .

To sum up the above description, it may be understood that the first sub-loss function corresponds to $$L_{n}^{1}$$ in Equation 3 and the second sub-loss function corresponds to $$L_{n}^{2}$$ in Equation 3. That is, it may be understood that the operation process of the first sub-loss function corresponds to (a) of FIG. 3 and the operation process of the second sub-loss function corresponds to (b) of FIG. 3.

Meanwhile, the loss function for matching the first and second operation functions in step S120 may further include a third sub-loss function for calculating the difference between the output of the first neural network model that has received medical data and the output of the second neural network model that has received medical data. When the first sub-loss function and the second sub-loss function are functions for matching the neural network blocks included in the individual neural network models from a detailed perspective, the third sub-loss function may be understood as a function for matching the neural network models from an overall model perspective.

The third sub-loss function may include a transformation function for matching the dimension of the output of the first neural network model that has received medical data to the dimension of the output of the second neural network model that has received medical data. As with the first and second sub-loss functions, when there is a difference in the type of neural network between the first and second neural network models, there is inevitably a difference in dimension between the pieces of data obtained from respective operation processes, so that the third sub-loss function may include a transformation function to solve the dimensional difference problem. For example, referring to (c) of FIG. 3, the transformation function included in the third sub-loss function may be used for an operation of transforming the dimension of the output of the first operation function corresponding to the final neural network block into the dimension of the output of the second operation function corresponding to the final neural network block, as in $h_n^{f \rightarrow g}$ .

To sum up the above description, it may be understood that the third sub-loss function corresponds to $L_n^3$ in Equation 4 and the operation process of the third sub-loss function corresponds to (c) in FIG. 3. Furthermore, the loss function for matching the first and second operation functions in step S120 may be represented by a combination of the first sub-loss function, the second sub-loss function, and the third loss function. Although the simple sum of the three sub-loss functions is shown in Equation 5, the sub-loss functions may be combined based on various operations such as a weighted sum and a multiplication as well as a simple sum.

The computing device 100 may fine-tune the second neural network model, trained on the inductive bias of the first neural network model through step S120, based on the medical data in step S130. In this case, fine tuning may be understood as a process of training the second neural network model while keeping the weight of the second neural network model close to the weight in the state in which training based on the first neural network model in step S120 has been completed. For example, in order for the second neural network model to be more optimized for the task of predicting arrhythmia among chronic diseases, the computing device 100 may fine-tune the second neural network model by inputting electrocardiogram data related to the prediction of arrhythmia to the second neural network model for which step S120 has been completed. Additionally, in order for the second neural network model to be more optimized for the task of monitoring heart rate changes of a patient hospitalized an intensive care unit, the computing device 100 may fine-tune the second neural network model by inputting the electronic health records data of a critically ill patient to the second neural network model for which step S120 has been completed. The fine tuning of the present disclosure is not limited to the above-described example, but may be performed in various manners within the range understandable by those skilled in the art based on the content of the present disclosure.

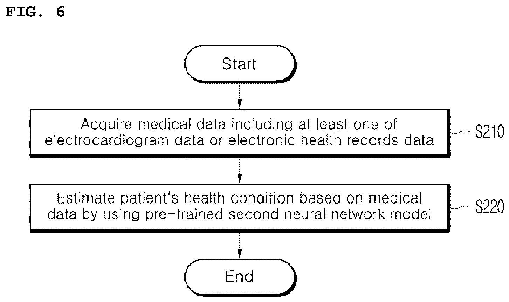

FIG. 6 is a flowchart showing the inference method of a deep learning model according to an embodiment of the present disclosure.

Referring to FIG. 6, the computing device 100 according to an embodiment of the present disclosure may acquire medical data including at least one of electrocardiogram data or electronic health records data in step S210. For example, when the computing device 100 is a server or client of a cloud system, the computing device 100 may receive at least one of the electrocardiogram data or electronic health records data of an inference target through communication with a database in a hospital environment. When the computing device 100 is a database within a hospital environment, the computing device 100 may generate at least one of the electrocardiogram data or electronic health records data of an inference target through communication with an electrocardiogram measurement device within the hospital environment.

The computing device 100 may estimate a person's health condition based on the medical data using the pre-trained second neural network model in step S220. The computing device 100 may predict the occurrence, change trend, degree of risk, and/or the like of the person's disease by inputting the at least one of the electrocardiogram data or the electronic health records data, acquired through step S210, to the pre-trained second neural network model. In this case, the second neural network model may have been trained through operations of matching first operation functions corresponding to the neural network blocks included in the first neural network model and second neural network functions corresponding to the neural network blocks included in the second neural network model based on the pre-trained first neural network model. Since the pre-training of the second neural network model corresponds to the description of FIG. 6 given above, a detailed description thereof will be omitted below.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

**Claims**

1. A method of training a deep learning model based on medical data, the method being performed by a computing device including at least one processor, the method comprising:

    training a first neural network model based on medical data; and
    training a second neural network model based on the trained first neural network model by matching a first operation function representative of a neural network block included in the trained first neural network model and a second operation function representative of a neural network block included in the second neural network model.

2. The method of claim 1, wherein training the second neural network model based on the trained first neural network model by matching the first operation function representative of the neural network block included in the trained first neural network model and the second operation function representative of the neural network block included in the second neural network model comprises training the second neural network model based on the trained first neural network model by using a loss function for matching at least one of inputs or dimensions between the first and second operation functions.

3. The method of claim 2, wherein the loss function comprises:

a first sub-loss function adapted to use an output of a first operation function corresponding to an (n-1)-th (n is a natural number) neural network block included in the first neural network model as an input variable; and a second sub-loss function adapted to use an output of a second operation function corresponding to an (n-1)-th neural network block included in the second neural network model as an input variable; wherein each of the first and second sub-loss functions is a function for calculating a difference between an output of a first operation function corresponding to an n-th neural network block included in the first neural network model and an output of a second operation function corresponding to an n-th neural network block included in the second neural network model.

4. The method of claim 3, wherein each of the first and second sub-loss functions comprises a transformation function for matching dimensions of the first operation function corresponding to the n-th neural network block and the second operation function corresponding to the n-th neural network block.

5. The method of claim 4, wherein the transformation function comprises:

a first sub-transformation function for linearly transforming input variables of the transformation function in a temporal direction; and a second sub-transformation function for linearly transforming input variables of the transformation function in a feature dimension.

6. The method of claim 4, wherein the transformation function included in the first sub-loss function is a function for:

matching a dimension of the output of the first operation function corresponding to the (n-1)-th neural network block to a dimension of the input of the second operation function corresponding to the n-th neural network block; and matching a dimension of the output of the second calculation function corresponding to the n-th neural network block to a dimension of the output of the first calculation function corresponding to the n-th neural network block.

7. The method of claim 4, wherein the transformation function included in the second sub-loss function is a function for:

matching a dimension of the output of the second operation function corresponding to the (n-1)-th neural network block to a dimension of the input of the first operation function corresponding to the n-th neural network block; and matching a dimension of the output of the first operation function corresponding to the n-th neural network block to a dimension of the output of the second operation function corresponding to the n-th neural network block.

8. The method of claim 2, wherein the loss function further comprises a third sub-loss function for calculating a difference between an output of the first neural network model having received the medical data and an output of the second neural network model having received the medical data.

9. The method of claim 8, wherein the third sub-loss function comprises a transformation function for matching the dimension of the output of the first neural network model having received the medical data to the dimension of the output of the second neural network model having received the medical data.

10. The method of claim 1, further comprising fine-tuning the second neural network model based on the medical data; wherein the fine-tuning is training the second neural network model while maintaining a weight of the second neural network model close to a weight in a state in which training based on the trained first neural network model has been completed.

11. The method of claim 1, wherein:

the first neural network model comprises at least one of a convolutional neural network or a recurrent neural network; and
the second neural network model comprises a self-attention-based neural network.

12. The method of claim 1, wherein the medical data comprises at least one of electrocardiogram data or electronic health records (EHR) data.

13. An inference method of a deep learning model based on medical data, the inference method being performed by a computing device including at least one processor, the inference method comprising:

acquiring medical data including at least one of electrocardiogram data or electronic health records (EHR) data; and
estimating a person's health condition based on the medical data by using a second neural network model;
wherein the second neural network model has been trained through operations for matching a first operation function corresponding to a neural network block included in a pre-trained first neural network model and a second operation function corresponding to a neural network block included in the second neural network model based on the pre-trained first neural network model.

14. A computer program stored in a computer-readable storage medium, the computer program performing operations for operations for training a deep learning model based on medical date when executed on one or more processors, wherein the operations comprise operations of:

training a first neural network model based on medical data; and
training a second neural network model based on the trained first neural network model by matching a first operation function representative of a neural network block included in the trained first neural network model and a second operation function representative of a neural network block included in the second neural network model.

15. A computing device for training a deep learning model based on medical data, the computing device comprising:

a processor comprising at least one core;
memory comprising program codes that are executable on the processor; and
a network unit configured to acquire medical data;
wherein the processor:

trains a first neural network model based on medical data; and
trains a second neural network model based on the trained first neural network model by matching a first operation function representative of a neural network block included in the trained first neural network model and a second operation function representative of a neural network block included in the second neural network model.

FIG. 1

100

Processor — 110

Memory — 120

Network Unit — 130

**FIG. 2**

FIG. 3

FIG. 4

(a)

(b)

FIG. 5

Start

Train first neural network model based
on medical data — S110

Train second neural network model by matching
operation functions representative of neural network
blocks between first and second neural network
models — S120

Fine-tune second neural network model — S130

End

FIG. 6

```
                          ┌─────────────┐
                          │    Start    │
                          └─────────────┘
                                 │
                                 ▼
          ┌───────────────────────────────────────────────┐
          │    Acquire medical data including at least one of │ ～ S210
          │  electrocardiogram data or electronic health records data │
          └───────────────────────────────────────────────┘
                                 │
                                 ▼
          ┌───────────────────────────────────────────────┐
          │   Estimate patient's health condition based on medical │ ～ S220
          │  data by using pre-trained second neural network model │
          └───────────────────────────────────────────────┘
                                 │
                                 ▼
                          ┌─────────────┐
                          │     End     │
                          └─────────────┘
```

<div align="center">**INTERNATIONAL SEARCH REPORT**</div>

| International application No. |
|---|
| **PCT/KR2022/013833** |

| A. | CLASSIFICATION OF SUBJECT MATTER |
|---|---|

**G16H 50/70**(2018.01)i; **G16H 50/50**(2018.01)i; **G16H 50/20**(2018.01)i; **G16H 10/60**(2018.01)i; **A61B 5/28**(2021.01)i; **G06N 3/08**(2006.01)i

According to International Patent Classification (IPC) or to both national classification and IPC

| B. | FIELDS SEARCHED |
|---|---|

Minimum documentation searched (classification system followed by classification symbols)

G16H 50/70(2018.01); G06N 20/00(2019.01); G06N 3/02(2006.01); G06N 3/04(2006.01); G06N 3/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 딥러닝(deep learning), 블록(block), 미세조정(fine tuning), 의료데이터(medical data), 손실함수(loss function)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2020-0052453 A (SAMSUNG SDS CO., LTD.) 15 May 2020 (2020-05-15)<br>See paragraphs [0006]-[0011]. | 1-2,14-15 |
| Y | | 10-13 |
| A | | 3-9 |
| Y | KR 10-2021-0068713 A (PDXEN) 10 June 2021 (2021-06-10)<br>See paragraphs [0017]-[0071]. | 10-13 |
| A | KR 10-2020-0045128 A (SAMSUNG ELECTRONICS CO., LTD.) 04 May 2020 (2020-05-04)<br>See paragraphs [0026]-[0071]; and figures 1-6. | 1-15 |
| A | KR 10-2021-0035381 A (SAMSUNG SDS CO., LTD. et al.) 01 April 2021 (2021-04-01)<br>See paragraphs [0047]-[0105]; and figures 2-8. | 1-15 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **02 January 2023** | **02 January 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2022/013833** |

**C.      DOCUMENTS CONSIDERED TO BE RELEVANT**

| Category* | Citation of document, with indication, where appropriate, of the relevant passages | Relevant to claim No. |
| --- | --- | --- |
| A | KR 10-2232138 B1 (A.I.MATICS INC.) 25 March 2021 (2021-03-25)<br>See paragraphs [0009]-[0090]; and figures 1-8. | 1-15 |

Form PCT/ISA/210 (second sheet) (July 2022)

INTERNATIONAL SEARCH REPORT
Information on patent family members

International application No.

**PCT/KR2022/013833**

| Patent document cited in search report | | | Publication date (day/month/year) | Patent family member(s) | | | Publication date (day/month/year) |
|---|---|---|---|---|---|---|---|
| KR | 10-2020-0052453 | A | 15 May 2020 | US | 2020-0134455 | A1 | 30 April 2020 |
| KR | 10-2021-0068713 | A | 10 June 2021 | None | | | |
| KR | 10-2020-0045128 | A | 04 May 2020 | US | 2020-0125927 | A1 | 23 April 2020 |
| KR | 10-2021-0035381 | A | 01 April 2021 | US | 11308608 | B2 | 19 April 2022 |
| | | | | US | 2021-0090247 | A1 | 25 March 2021 |
| KR | 10-2232138 | B1 | 25 March 2021 | US | 2022-0156596 | A1 | 19 May 2022 |
| | | | | WO | 2022-108015 | A1 | 27 May 2022 |

Form PCT/ISA/210 (patent family annex) (July 2022)